# EUROPEAN PATENT APPLICATION

(11) **EP 0 826 653 A1**
(43) Date of publication of application: **04.03.1998**
(21) Application number: 96113909.4
(22) Date of filing: 30.08.1996
(51) Int. Cl.: C07C 2/66, C07C 15/073

(54) **Process for the alkylation of aromatic hydrocarbons with recycle of reactor effluent**

(71) Applicant: FINA TECHNOLOGY, INC., Dallas, Texas 75206 (US)
(72) Inventor: Merrill, James T., Houston, Texas 77059 (US); Butler, James R., Katy, Texas 77449 (US)
(74) Representative: Detrait, Jean-Claude

(57) **Abstract**

A process for the production of an alkylated aromatic hydrocarbon such as ethylbenzene by alkylation of benzene with an olefin such ethylene, propylene, 1-butene or isobutene with a catalyst in an alkylation reactor under liquid or gas phase conditions includes alkylating benzene with an olefin in a reaction zone at a temperature of from about 175°C. to about 500°C. and a pressure of from about 200 psi to about 800 psi, withdrawing a reactor effluent stream and separating the reactor effluent stream into a product stream and a recycle stream each containing ethylbenzene and unreacted benzene and directing the recycle stream containing ethylbenzene and unreacted benzene back to the reaction zone wherein the ratio of the liquid volume of the recycle stream to the volume of fresh benzene feed is from 0.1:1 to 50:1.

## Description

### TECHNICAL FIELD

The present invention relates to a process for alkylating aromatic hydrocarbons such as benzene with an alkylating agent such as ethylene using direct recycle of a portion of the reactor effluent without purification of the recycled aromatic hydrocarbon.

### BACKGROUND OF THE INVENTION

Ethylbenzene is used primarily for the production of styrene monomer obtained through dehydrogenation. A large portion of ethylbenzene currently produced is obtained by alkylation of benzene with ethylene under a variety of alkylation conditions. One type of alkylation process involves vapor phase reactions in which benzene is alkylated with an olefin such as ethylene under high temperatures and pressures utilizing a catalyst in a multiple bed reactor. Another type of alkylation process involves liquid phase alkylation of benzene with ethylene, also utilizing a catalyst. In either case benzene, in excess of a stoichiometric amount, is used in the process for temperature control since the reaction is highly exothermic. A second reason for using a stoichiometric excess of benzene is for reaction efficiency. Dilution of the ethylene supplied to the process increases the selectivity of the reaction for the production of ethylbenzene whereas higher concentrations of ethylene tend to result in the production of more undesired oligomers. Thus, in conventional processes for the alkylation of benzene with ethylene, a stoichiometric excess of benzene is supplied to the alkylation reactor, producing a reactor effluent comprising in large part unreacted benzene.

It has long been believed that it was necessary to process the entire reactor effluent stream after a single pass through the reactor to separate and purify the benzene in the reactor effluent in order to maintain process control. Therefore, in conventional commercial scale alkylation processes in which benzene is alkylated with ethylene, the entire reactor effluent is subjected to successive distillations to separate benzene, ethylbenzene, polyethylbenzenes and heavy residues into separate streams. A significant capital expenditure in equipment is required in order to provide the processing capability required to treat the entire reactor effluent stream in this manner. Additionally, the process of separating benzene from the alkylation reactor effluent consumes a significant amount of energy.

Moreover, in conventional commercial scale processes in which the feed stream containing benzene and ethylene makes a single pass through the reactor, the concentration of ethylbenzene, the desired product, in the reactor effluent is limited. The limiting factor is the amount of ethylene in the reactor feed since, as noted above, a stoichiometric excess of benzene is supplied to the alkylation reactor. Since the single pass mode of operation necessarily limits the concentration of the desired ethylbenzene product in the reactor effluent, a larger volume effluent must be processed to obtain a desired production level.

In connection with the foregoing, it has been discovered that a process for the vapor or liquid phase alkylation of benzene with an olefin such as ethylene may include direct recycle of a large portion of the reactor effluent, thereby providing the potential for decreased energy consumption and capital costs.

### SUMMARY OF THE INVENTION

The present invention provides a method for alkylation of an aromatic hydrocarbon with an olefin, such as the alkylation of benzene with ethylene using a conventional alkylation catalyst to produce ethylbenzene under either gas or liquid phase reaction conditions. The process of the present invention provides for the production of alkylated hydrocarbons such as ethylbenzene with reduced energy consumption and capital cost as compared to conventional processes.

In a first embodiment of the process of the present invention, an aromatic hydrocarbon such as benzene is alkylated with an olefin such as ethylene using a conventional alkylation catalyst to produce an alkylated aromatic hydrocarbon such as ethylbenzene under liquid phase conditions. In this embodiment, the alkylation reaction takes place at temperatures from about 175°C. to about 250°C. and at pressures from about 600 psi to about 800 psi. Reactor effluent is separated into a product stream and a recycle stream which is recycled to the reactor inlet. The ratio of the volume of the recycled reactor effluent to the volume of fresh benzene feed is maintained from 0.1:1 to 50:1, or more preferably from 4:1 to about 25:1. The liquid volume of the recycle stream may be as large or larger than the volume of the product stream. Under these conditions the process of the present invention provides a method for alkylating benzene with ethylene to produce ethylbenzene with very low concentrations of xylenes.

In a second embodiment of the process of the present invention, benzene is alkylated with ethylene using a conventional alkylation catalyst to produce ethylbenzene under gas phase conditions. The gas phase alkylation reaction takes place with a temperature range of from about 350°C. to about 500°C. and a pressure range of from about 200 psi to about 500 psi. The ratio of the liquid volume of the recycled stream to the volume of fresh benzene feed is from 0.1:1 to 50:1, depending on operating conditions, and the molar ratio of fresh benzene to ethylene in the feed stream is in the range from about 0.5:1 to 15:1.

Although the process of the present invention is described herein in connection with the alkylation of benzene with ethylene, it will be understood that the process may be applied to the alkylation of benzene with other olefins such as propylene, 1-butene and isobutene.

### BRIEF DESCRIPTION OF THE DRAWINGS

Without limiting the scope of the appended claims which are intended to be cover variations and modifications within the spirit and scope of the invention, further aspects of the invention and its advantages may be appreciated by reference to the following detailed description taken in conjunction with the drawings in which:
FIGURE 1 is a schematic illustration of a first embodiment of the process of the present invention in which benzene is alkylated with an olefin such as ethylene under liquid phase conditions;
FIGURE 2 is a schematic illustration of a second embodiment of the process of the present invention in which benzene is alkylated with an olefin such as ethylene under liquid phase conditions in a downflow mode; and
FIGURE 3 is a schematic illustration of a third embodiment of the process of the present invention in which benzene is alkylated with an olefin such as ethylene under gas phase conditions.

### DETAILED DESCRIPTION

The process of the present invention can be carried out using a variety of process equipment, including a reactor vessel that defines a reaction zone containing catalyst material and aromatic hydrocarbons. Either single or multiple catalyst beds can be employed in the reaction zone. The olefin and benzene reactants may be mixed and preheated prior to introduction to the reaction zone which may consist of one or more catalyst beds where the reactants contact the catalyst under reaction conditions. If multiple catalyst beds are employed, the olefin and benzene may be injected into the reactor at locations corresponding to the different catalyst beds for the purposes of process control. Reactor effluent containing reaction products and unreacted benzene are withdrawn from the reaction zone after a controlled residence time and a portion of the reaction products are collected and separated by conventional techniques. The remainder of the reaction products along with unreacted benzene, the recycle stream, is recycled to the reaction zone. The volume of recycled reaction products and unreacted benzene may be as large or larger than the volume of the product stream and the concentration of alkylated aromatic hydrocarbon and unreacted aromatic hydrocarbon in the recycle stream is substantial equal to that of the product stream.

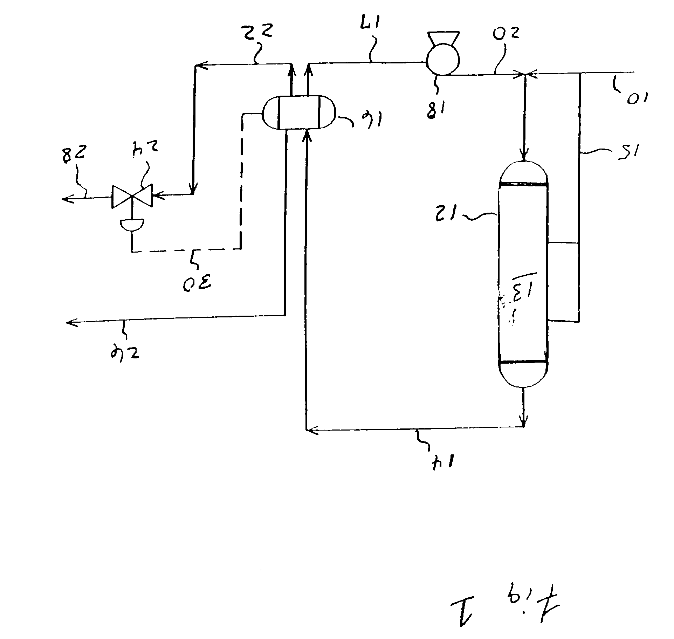

Turning now to FIGURE 1, a first embodiment of the process of the present invention is schematically illustrated. A feed stream containing benzene and an alkylating agent such as ethylene are injected into an alkylation reactor 12 through line 10 where the reactants are brought into contact with a catalyst in catalyst zone 13 under liquid phase conditions. As illustrated, the reactor 12 is operated in an upflow mode. Since the alkylation reaction is highly exothermic, the reactants may be injected at multiple locations in the reactor through line 15 for process control purposes. The alkylation reaction takes place at temperatures from about 175°C. to about 250°C. and at pressures from about 600 psi to about 800 psi. Preferably, the reaction conditions are maintained at approximately 200°C. and about 600 psi.

An effluent stream 14 from the reactor 10 is directed to a separation vessel 16. A product stream is withdrawn from separation vessel 16 through line 22 and control valve 24 and is directed through line 28 to one or more fractionating columns (not shown) where the ethylbenzene product is separated from the other components in the product stream. The liquid level in separation vessel 16 is maintained through the actuation of control valve 24 effected by control loop 30 utilizing conventional instrumentation and controls. Gaseous components of the effluent stream are vented through line 26 for collection and processing.

A recycle stream is withdrawn from vessel 16 through line 17 and recycle pump 18 which directs the recycle stream through line 20 for mixing with the feed stream and injection into reactor 12. The recycle ratio, i.e. the ratio of the volume of the recycled liquid stream to the volume of fresh benzene feed may vary from 0.1:1 to 50:1, or more preferably from 4:1 to about 25:1, depending on operating conditions. Weight hourly space velocities are preferably in the range from about 20 to 150.

The molar ratio of fresh benzene to ethylene in the feed stream may be varied from 0.5:1 to 15:1, more preferably from 0.5:1 to 1.5:1 depending upon process conditions. It has been observed in pilot scale operations that steady state concentrations of ethylbenzene as high as 42 wt% in the reactor effluent have been achieved with a fresh benzene:ethylene ratio of 0.85:1. With a fresh benzene:ethylene ratio of 1.5:1, a steady state ethylbenzene concentration in the reactor effluent of 28 wt% was reached in a pilot scale operation.

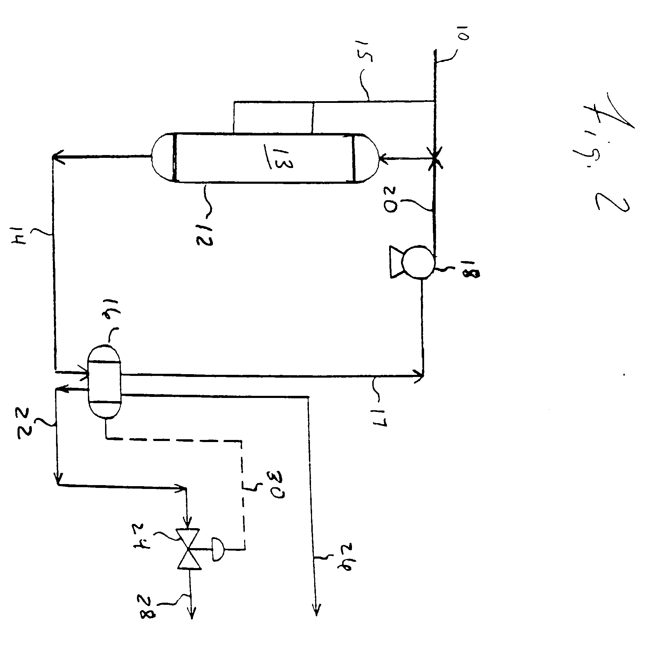

Referring now to FIGURE 2, an alternate embodiment of the process schematically illustrated in FIGURE 1 is shown in which the reactor is operated in a downflow mode. The process conditions and parameters are the same as described in connection with FIGURE 1. A feed stream containing benzene and an alkylating agent such as ethylene are injected into an alkylation reactor 12 through line 10 where the reactants are brought into contact with a catalyst in catalyst zone 13 under liquid phase conditions. Since the alkylation reaction is highly exothermic, the reactants may be injected at multiple locations in the reactor through line 15 for process control purposes.

An effluent stream 14 from the reactor 10 is directed to a separation vessel 16. A product stream is withdrawn from separation vessel 16 through line 22 and control valve 24 and is directed through line 28 to one or more fractionating columns (not shown) where the ethylbenzene product is separated from the other components in the product stream. The liquid level in separation vessel 16 is maintained through the actuation of control valve 24 effected by control loop 30 utilizing conventional instrumentation and controls. Gaseous components of the effluent stream are vented through line 26 for collection and processing.

A recycle stream is withdrawn from vessel 16 through line 17 and recycle pump 18 which directs the recycle stream through line 20 for mixing with the feed stream and injection into reactor 12. The recycle ratio, i.e. the ratio of the volume of the recycled liquid stream to the volume of fresh benzene feed may vary from 0.1:1 to 50:1, or more preferably from 4:1 to about 25:1, depending on operating conditions. Weight hourly space velocities are preferably in the range from about 20 to 150. The molar ratio of fresh benzene to ethylene in the feed stream may be varied from 0.5:1 to 15:1, more preferably from 0.5:1 to 1.5:1 depending upon process conditions.

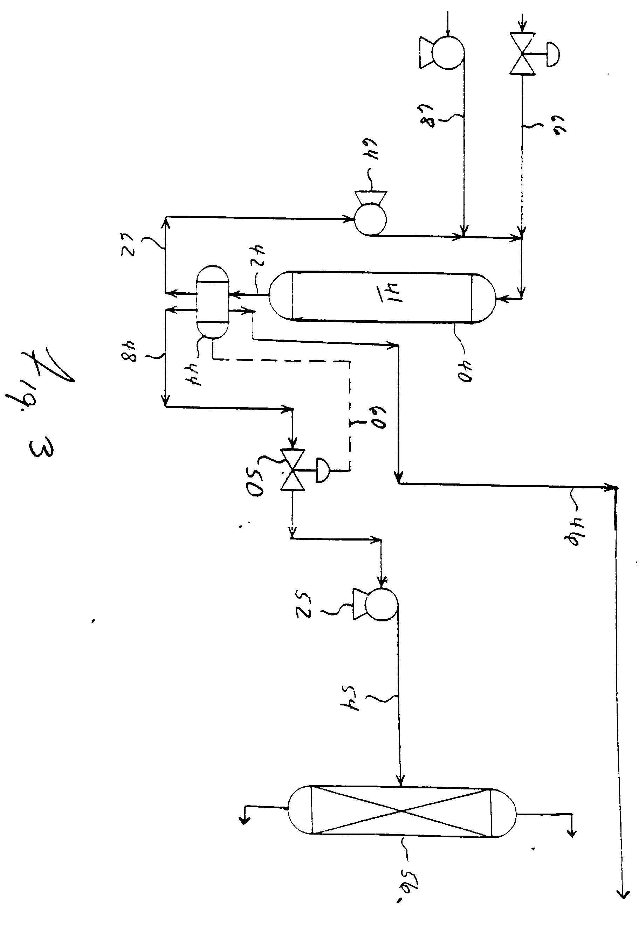

Turning now to FIGURE 3, a second embodiment of the process of the present invention is schematically illustrated. Alkylation reactor 40 is operated under gas phase alkylation reaction conditions with recycle for the production of ethylbenzene from benzene and ethylene. The reactor effluent is withdrawn from the reactor 40 through line 42 into separation vessel 44 where gaseous components of the effluent stream are vented through line 46 for collection and processing. A product stream is withdrawn from the separation vessel 44 through line 48 and control valve 50. Distillation pump 52 pumps the product stream through line 54 to one or more distillation columns 56 for separation of the desired ethylbenzene product from the other components contained in the product stream. Control valve 50 controls the fluid level in separation vessel 44 through the operation of control loop 60 using conventional instrumentation and controls.

A recycle stream is withdrawn from separation vessel 44 through recycle line 62. Recycle pump 64 directs the recycle stream to the top of reactor 40 for reintroduction into the reaction zone 41 which may contain one or more catalyst beds. Ethylene and fresh benzene are introduced into the recycle line 62 via feed lines 66 and 68. In practice, the recycle stream and the fresh benzene and ethylene may be injected at multiple locations in the reactor for process control purposes. The catalyst used in the process of the present invention is selective to the production of ethylbenzene in a temperature range of from about 175°C. to about 500°C. and a pressure range of from about 200 psi to about 800 psi. Although reaction conditions employing temperatures from about 250°C. to about 500°C. may be utilized under gas phase conditions, more preferably the reaction is conducted within a range of from about 300°C. to about 475°C.

Under gas phase conditions the recycle ratio, i.e. the ratio of the liquid volume of the recycled stream to the volume of fresh benzene feed may vary from 0.1:1 to 50:1, depending on operating conditions. In pilot scale operations, under gas phase reaction conditions, a recycle ratio of about 4.5:1 with a fresh benzene:ethylene ratio of approximately 6:1 has been found to be maintainable. In commercial scale operations it is anticipated that higher recycle ratios will prove to advantageous. Preferably the recycle ratio and ratio of fresh benzene to ethylene will be adjusted to achieve a ethylbenzene concentration in the reactor effluent of 30% or higher. Weight hourly space velocities are preferably in the range from about 20 to 150.

The molar ratio of fresh benzene to ethylene in the feed stream may be varied from 0.5:1 to 15:1, more preferably from 0.5:1 to 10:1 depending upon process conditions.

The process of the present invention may be further illustrated by the following examples which are not to be construed as limiting the scope of the invention as hereinafter claimed.

### EXAMPLE 1

Liquid phase alkylation of benzene with ethylene is conducted under reaction conditions as follows. Five milliliters of a conventional low sodium alkylation catalyst having a particle size distribution of between 20 and 40 mesh is introduced into a laboratory scale reactor. The catalyst is heated under nitrogen flow to 150°C at ambient pressure to dry the catalyst. The temperature is increased to 200°C as measured at the reactor inlet and a feed stream of benzene and ethylene is introduced into the reactor. The fresh benzene:ethylene mole ratio in the feed stream is 1.5:1. The reactor pressure is maintained at 600 psig and the reactor is operated in an up-flow mode. Average reactor temperatures range between about 190°C and 220°C. Liquid hourly space velocities range from 65 to 75 hr^{-1.}

The test is run for a period of 21 days and selected values for the fresh benzene feed rate, recycle rate and concentration of ethylbenzene in the product stream are recorded in Table 1 below:

**TABLE 1**

| Test Duration (days) | Fresh Benzene Feed (ml/min) | Liquid Recycle (mil/min) | Ethylbenzene in Product (wt%) |
|---|---|---|---|
| 9 | 0.33 | 5.37 | 24.17 |
| 10 | 0.38 | 5.53 | 25.61 |
| 11 | 0.38 | 5.65 | 26.20 |
| 12 | 0.38 | 5.65 | 26.72 |
| 13 | 0.38 | 5.61 | 26.98 |
| 14 | 0.38 | 5.43 | 27.69 |
| 15 | 0.38 | 5.53 | 27.67 |
| 16 | 0.38 | 5.44 | 28.19 |
| 17 | 0.38 | 5.53 | 28.36 |
| 18 | 0.39 | 5.45 | 28.92 |
| 19 | 0.38 | 5.46 | 29.01 |
| 20 | 0.38 | 5.38 | 28.75 |
| 21 | 0.37 | 5.52 | 29.09 |

Only trace amounts of xylenes, on the order of 0.001 wt% are measured in the liquid product stream.

### EXAMPLE 2

Liquid phase alkylation of benzene with ethylene is conducted under the same reaction conditions as in Example 1, except that the fresh benzene:ethylene mole ratio in the feed stream is maintained at 0.85:1. The test is run for 21 days and the fresh benzene feed rate, recycle rate and concentration of ethylbenzene in the product stream are recorded in Table 2 below:

**TABLE 2**

| Test Duration (days) | Fresh Benzene Feed (ml/min) | Liquid Recycle (mil/min) | Ethylbenzene in Product (wt%) |
|---|---|---|---|
| 1 | 0.35 | 5.87 | 7.48 |
| 2 | 0.36 | 5.85 | 13.06 |
| 3 | 0.35 | 5.82 | 16.41 |
| 4 | 0.36 | 5.84 | 18.58 |
| 5 | 0.22 | 5.47 | 21.80 |
| 6 | 0.22 | 5.56 | 21.93 |
| 7 | 0.21 | 5.55 | 27.34 |
| 14 | 0.20 | 5.55 | 37.38 |
| 15 | 0.22 | 5.58 | 39.82 |
| 16 | 0.21 | 5.58 | 40.82 |
| 17 | 0.23 | 5.53 | 41.42 |
| 18 | 0.21 | 5.56 | 42.58 |
| 19 | 0.22 | 5.56 | 42.70 |
| 20 | 0.20 | 5.71 | 43.78 |
| 21 | 0.20 | 5.52 | 41.91 |

### EXAMPLE 3

Gas phase alkylation of benzene with ethylene is conducted under reaction conditions as follows. Twenty milliliters of a conventional low sodium alkylation catalyst having a particle size distribution of between 20 and 40 mesh is introduced into a laboratory scale reactor. The catalyst is heated under nitrogen flow to 150°C at ambient pressure to dry the catalyst. The temperature is increased to 200°C as measured at the reactor inlet and a feed stream of benzene and ethylene is introduced into the reactor. The fresh benzene:ethylene mole ratio in the feed stream is maintained at approximately 6:1. The reactor pressure is maintained at approximately 300 psig and the reactor is operated in a down-flow mode. Average reactor temperatures range from about 400°C to about 410°C. Liquid hourly space velocities range between 60 and 70 hr⁻¹.

The test is run for a period of 85 days. The fresh benzene feed rate is maintained at approximately 4 ml./min. Ethylene is fed at a rate to maintain a molar ratio of fresh benzene:ethylene of approximately 6.5. The recycle ratio is approximately 4.5 and the concentration of ethylene in the reactor effluent reaches a steady state value of about 18 wt%.

As can readily be observed from the foregoing Examples, alkylation of benzene with ethylene under gas or liquid phase conditions using a conventional alkylation catalyst to produce ethylbenzene can be advantageously accomplished with recycle of the reactor effluent to increase the concentration of ethylbenzene in the product stream. Increasing the concentration of ethylbenzene in the product stream reduces the volume of reactor effluent that must be processed in order to achieve a desired production rate of the ethylbenzene product. The increase in ethylbenzene concentration in the product stream afforded by the process of the present invention provides the potential for reduced energy and capital.

Although the process of the present invention is described above primarily in connection with the alkylation of benzene with ethylene, the scope of the invention and the appended claims is not thereby limited, it being understood that the process may be applied to the alkylation of benzene with other olefins such as propylene, 1-butene and isobutene.

While the invention has been described in connection with the foregoing examples and drawings, it will be appreciated by the those skilled in the art that the invention is subject to variations and modifications which fall within the scope of the appended claims and which are intended to be covered thereby.

## Claims

1. A process for the alkylation of an aromatic hydrocarbon with an alkylating agent utilizing a catalyst in an alkylation reactor including a reaction zone comprising at least one catalyst bed, the process comprising the steps of:
introducing a fresh aromatic hydrocarbon, an alkylating agent and a recycle stream into the reaction zone under reaction conditions;
alkylating the aromatic hydrocarbon with the alkylating agent in the reaction zone;
withdrawing a reactor effluent stream containing alkylated aromatic hydrocarbon and unreacted aromatic hydrocarbon from the reactor zone;
separating the reactor effluent stream into a product stream and the recycle stream, the product stream and recycle stream each containing alkylated aromatic hydrocarbon and unreacted aromatic hydrocarbon; and
directing the recycle stream containing alkylated aromatic hydrocarbon and unreacted aromatic hydrocarbon back to the reaction zone.

2. The process of Claim 1, wherein the aromatic hydrocarbon is benzene.

3. The process of Claim 1 wherein the alkylating agent is an olefin.

4. The process of Claim 3 wherein the olefin is selected from the group consisting of ethylene, propylene, 1-butene and isobutene.

5. The process of Claim 1 wherein the aromatic hydrocarbon is alkylated with an alkylating agent using a catalyst in a reaction zone under liquid phase reaction conditions.

6. The process of Claim 1 wherein the aromatic hydrocarbon is alkylated with an alkylating agent using a catalyst in a reaction zone under gas phase reaction conditions.

7. The process of Claim 1 wherein the ratio of the liquid volume of the recycled stream to the volume of fresh aromatic hydrocarbon feed is from 0.1:1 to 50:1.

8. The process of Claim 1 wherein the concentration of alkylated aromatic hydrocarbon and unreacted aromatic hydrocarbon in the recycle stream is substantially equal to the concentration of alkylated aromatic hydrocarbon and unreacted aromatic hydrocarbon in the product stream.

9. The process of Claim 1 wherein the reaction conditions include a temperature of from about 175°C. to about 500°C. and a pressure of from about 200 psi to about 800 psi.

10. A process for the alkylation of benzene with an olefin utilizing a catalyst in an alkylation reactor including a reaction zone comprising at least one catalyst bed, the process comprising the steps of:
introducing fresh benzene, an olefin and a recycle stream into the reaction zone under reaction conditions;
alkylating benzene with the olefin in the reaction zone;
withdrawing a reactor effluent stream containing alkylated benzene and unreacted benzene from the reactor;
separating the reactor effluent stream into a product stream and the recycle stream, the product stream and recycle stream each containing alkylated benzene and unreacted benzene; and
directing the recycle stream containing alkylated benzene and unreacted benzene back to the reaction zone.

11. The process of Claim 10 wherein the olefin is selected from the group consisting of ethylene, propylene, 1-butene and isobutene.

12. The process of Claim 10 wherein benzene is alkylated with ethylene utilizing a catalyst in a reaction zone under liquid phase reaction conditions.

13. The process of Claim 10 wherein benzene is alkylated with ethylene utilizing a catalyst in a reaction zone under gas phase reaction conditions.

14. The process of Claim 10 wherein the ratio of the liquid volume of the recycled stream to the volume of fresh benzene feed is from 0.1:1 to 50:1.

15. The process of Claim 10 wherein the concentration of alkylated benzene and unreacted benzene in the recycle stream is substantially equal to the concentration of alkylated benzene and unreacted benzene in the product stream.

16. The process of Claim 10 wherein the reaction conditions include a temperature of from about 175°C. to about 500°C. and a pressure of from about 200 psi to about 800 psi.

17. A process for the production of ethylbenzene by alkylation of benzene with ethylene with a catalyst in an alkylation reactor including a reaction zone comprising at least one catalyst bed, the process comprising the steps of:
introducing fresh benzene, ethylene and a recycle stream into the reaction zone;
alkylating benzene with ethylene in the reaction zone at a temperature of from about 175°C. to about 500°C. and a pressure of from about 200 psi to about 800 psi;
withdrawing a reactor effluent stream containing ethylbenzene and unreacted benzene from the reactor;
separating the reactor effluent stream into a product stream and the recycle stream, the product stream and recycle stream each containing ethylbenzene and unreacted benzene, the ratio of the liquid volume of the recycle stream to the volume of fresh benzene feed being from 0.1:1 to 50:1; and
directing the recycle stream containing ethylbenzene and unreacted benzene back to the reaction zone.

18. The process of Claim 17 wherein benzene is alkylated with ethylene using a catalyst in a reaction zone under liquid phase reaction conditions.

19. The process of Claim 17 wherein benzene is alkylated with ethylene using a catalyst in a reaction zone under gas phase reaction conditions.

20. The process of Claim 17 wherein the concentration of ethylbenzene and unreacted benzene in the recycle stream is substantially equal to the concentration of ethylbenzene and unreacted benzene in the product stream.
